# EUROPEAN PATENT APPLICATION

(11) **EP 0 834 572 A2**
(43) Date of publication of application: **08.04.1998**
(21) Application number: 97116590.7
(22) Date of filing: 24.09.1997
(51) Int. Cl.: C12N 15/87, C07K 14/47

(54) **Alpha, gamma-diaminobutyric acid (DAB) containing oligopeptide derivatives**

(30) Priority: 02.10.1996 EP 96115784
(71) Applicant: F. HOFFMANN-LA ROCHE AG, 4070 Basel (CH)
(72) Inventor: Legendre, Jean-Yves, 75013 Paris (FR); Supersaxo, Andreas, 4052 Basle (CH); Trzeciak, Arnold, 79650 Schopfheim (DE)
(74) Representative: Braun, Axel

(57) **Abstract**

alpha,gamma-Diaminobutyric acid (DAB) containing oligopeptide derivatives of formula

R'-NH-A I

wherein
- R': is a cell recognition agent, a membrane permeabilizing agent, a subcellular localization agent or a masking agent;
- A: is an oligopeptide devoid of one amino group and containing 1 to 200 amino acids, wherein at least one of the amino acid is alpha,gamma-diaminobutyric acids (DAB) and the terminal carboxyl group of the oligopeptide is converted into an amide, lower alkyl amide, di-lower alkyl amide or hydrazide
are useful as a carrier for the transfection of cells with a polynucleotide or any other anionic macromolecule.

## Description

Gene transfer technology has become a field of considerable interest. Introduction of an exogenous gene into a cell (i.e. transfection) bears many important scientific and medical applications, going from gene regulation and the production of recombinant proteins to gene therapy.

Viruses have evolved to bypass the different cellular barriers to gene transfer and have indeed become vectors of choice for transfection. Many viruses, including retrovirus, adenovirus or herpes virus, are now engineered to carry therapeutic genes and used in human clinical trials for gene therapy. However, there remains a risk of infectious and immunologic reaction and the large scale production of viruses is difficult and time consuming.

For these various reasons non viral systems have been developed to carry DNA into cells, e.g., the transfection technique based on a cationic lipid, the dioleoyloxypropyl trimethylammonium (Felgner et al., Proc. Natl., Acad. Sci. USA, 84, 7413-7417, 1987) commercialised as Lipofectin™. Since the discovery of this transfection technique, many more cationic lipids have been synthesised and some are commercially available as transfecting reagent for laboratory use: DOGS (Transfectam™), DOSPA (Lipofectamine™), DOTAP (DOTAP™).

Nevertheless, despite an important progress in the formulation of non-viral gene delivery systems, there remains a need for more efficient techniques, since the transfection efficiency of synthetic systems is usually below that of viral vectors. Furthermore, still many problems arise in vivo and the poor stability of the non-viral systems in biological fluids does not allow high and reproducible levels of transfection in vivo.

In accordance with the present invention, it has been found that alpha,gamma-diaminobutyric acid (DAB) containing oligopeptide derivatives bind polynucleotides and anionic macromolecules and can be used for transfecting cells.

Thus, in one aspect, the present invention relates to novel alpha,gamma-diaminobutyric acid (DAB) containing oligopeptide derivatives of formula

R'-NH-A I

wherein
- R': is a cell recognition agent, a membrane permeabilizing agent, a subcellular localization agent or a masking agent;
- A: is an oligopeptide devoid of one amino group and containing 1 to 200 amino acids, wherein at least one of the amino acid is alpha,gamma-diaminobutyric acids (DAB) and the terminal carboxyl group of the oligopeptide is converted into an amide, lower alkyl amide, di-lower alkyl amide or hydrazide.

The oligopeptides A may be linear or branched. The amino acids may belong to the L- or D- series or may be racemic. Compounds of formula I, wherein A is an oligopeptide devoid of one amino group containing 1 to 50 amino acids are preferred. Most preferred are compounds of formula I, wherein the oligopeptide A contains 1 to 20 amino acids.

Another preferred embodiment are compounds of formula I wherein the terminal carboxyl group of the oligopeptide A is converted into a hydrazide.

The "cell recognition agent" as used herein refers to a molecule capable of recognizing a component on the surface of a targeted cell. Cell recognition components include antibodies to cell surface antigens, ligands for cell surface receptors including those involved in receptor-mediated endocytosis, peptide hormones, etc. Specific ligands contemplated by this invention include carbohydrate ligands such as galactose, mannose, mannosyl 5-phosphate, fucose, sialic groups, N-acetylglucosamine or combinations of these groups as complex carbohydrates such as those found on glycolipids of the blood groups or on various secreted proteins. Other ligands include folate, biotin, various peptides that can interact with cell surface or intracellular receptors such as the chemoattractant peptide N-formyl-met-leu-phe (SEQ ID NO:2, WO-A-0 2397), peptides containing the arg-asp-gly sequence or cys-ser-gly-arg-glu-asp-val-trp (SEQ ID NO:3, ibid.) peptides, peptides that contain a cystine residue or that interact with cell surface protein such as the human immunodeficiency virus GP-120, and peptides that interact with CD-4. Other ligands include antibodies or antibody fragments such as those described by Hertler and Frankel (Hertler, A., and Frankel, A., J. Clin. Oncol. 7: 1932 (1989)). The specificity of the antibodies can be directed against a variety of epitopes that can be expressed on cell surfaces including histocompatibility macromolecules, autoimmune antigens, viral, parasitic or bacterial proteins. Other protein ligands include hormones such as growth hormone and insulin or protein growth factors such as GM-CSF, G-CSF, erythropoietin, epidermal growth factor, basic and acidic fibroblast growth factor, and the like. Other protein ligands include various cytokines that work through cell surface receptors such as interleukin 2, interleukin 1, interleukin 12, tumor necrosis factor, and suitable peptide fragments from such macromolecules.

In another embodiment, the cell recognition molecule is an integrin-binding peptide or derivatives thereof. In a more preferred embodiment the integrin-binding peptide is GGCRGDMFGCGG.

The "membrane permeabilizing agent" as used herein refers to a molecule that aids in the passage of a polynucleotide or anionic macromolecule across a membrane. Examples of membrane permeabilizing agents are melittin, hemolysin, mastoparan, bombolitin, crabrolin, pardaxin, gramicidin, alamethicin, apidaecin, bactenecin, cecropins, defensins, dermaseptin, indolicidin, magainins, bombinin, brevinin, esculentin, seminalplasmin and derivatives thereof (G. Saberwal and R. Nagaraj. BBA 1197:109-131 (1994)). Other membrane permeabilizing agents are peptides containing the amino-terminal amino acid sequence of the influenza virus hemagglutinin and synthetic derivatives thereof as described in the Journal of Biological Chemistry, Vol. 269 (17), 12918-12924 (1984) (Table I); or HIV and SIV fusion peptides and synthetic derivatives thereof as described in Biochemica et Biophysica Acta 1240, 95-100 (1995) (Fig. 1). Another class of cell permeabilizing agents are detergent molecules such as bile salts. Other examples of detergent molecules are sucrose monolaurate, n-octylglucoside and tocopheryl PEG 1000 succinate. However, other detergent molecules are also suitable.

In another embodiment the membrane permeabilizing agents is a conjugate between a membrane permeablilizing agent and a lipid. An example of such a conjugate is phe-glu-ala-ala-leu-ala-glu-ala-leu-ala-glu-ala-leu-ala with an NH₂-terminal myristic acid (C. Puyal et al. BBA 1195:259-266 (1994)).

The "subcellular localization agent" as used herein refers to a molecule capable of recognizing a subcellular component in a targeted cell. Particular subcellular components include the nucleus, ribosomes, mitochondria, and chloroplasts.

In a preferred embodiment of this invention, the subcellular-localization component is a nuclear-localization component. The nuclear-localization components include known peptides of defined amino acid sequences, and longer sequences containing these peptides. One known peptide sequence is the SV 40 large T antigen heptapeptide pro-lys-lys-lys-arg-lys-val (SEQ ID NO:1, WO-A-0 2397). Other peptides include the influenza virus nucleoprotein decapeptide ala-ala-phe-glu-asp-leu-arg-val-leu-ser (SEQ ID No:4, ibid.), and the adenovirus E1a protein segment lys-arg-pro-arg-pro (SEQ ID NO:5, ibid.). Other sequences may be discerned from Dingwall et al. (Dingwall, C., et al., TIBS 16:478 (1991)).

In another embodiment, the subcellular-localization component is a lysosomal-localization component. A known component for targeting the lysosome is a peptide containing the lys-phe-glu-arg-gln (SEQ ID NO:6, WO-A-0 2397) segment.

In yet another embodiment, the subcellular-localization component is a mitochondrial-localization component. A known component for targeting mitochondria is a peptide containing the sequence met-leu-ser-leu-arg-gln-ser-ile-arg-phe-phe-lys-pro-ala-thr-arg (SEQ ID NO:7, ibid.). However, other subcellular-localization components or agents are also suitable.

The "masking agent" as used herein refers to a molecule capable of masking all or part of the polynucleotide or anionic macromolecule, thereby increasing its circulatory half-life by inhibiting attack by degrading reagents present in circulation or by blocking uptake by the reticuloendothelial system. An example of such a masking agent is polyethylene glycol (PEG). The PEG may have a molecular weight of about 700 to 20,000 Daltons, preferably about 1800 to 6000 Daltons.

The compounds of formula I can be prepared by methods known in the art, for example, in analogy to the method described in Gene Therapy (1995) 2, 552-554. This invention relates thus to a process for preparing the novel compounds of formula I.

The invention further relates to the use of a compound of formula I as carrier for transfecting a cell with a polynucleotide or any other anionic macromolecule.

Examples of polynucleotides that can be transfected into cells by means of the novel compounds of this invention are deoxyribonucleic acids (DNA) and ribonucleic acids (RNA). Examples of anionic macromolecules other than polynucleotides are proteins, such as ribonucleoproteins and proteins used for immunisation, e.g. viral proteins.

Examples of DNA that can be transfected into cells by means of the novel compounds of this invention are plasmids and genes, especially those for which gene therapy protocols have been launched such as cystic fibrosis transmembrane regulator (CFTR), adenosine deaminase (ADA), thymidine kinase (tk) and HLA B7; as well as reporter genes such as beta-galactosidase, luciferase, chloramphenicol acetyl transferase and alpha-1 antitrypsin. Other examples of DNA are oligodeoxynucleotides and their analogues used as antisense, aptamer or "triple-helix" agents. Examples of RNA are ribozymes or oligoribonucleotides antisense molecules.

The nature of the cell which is to be transfected is not narrowly crucial. The cell can be a procaryotic or eucaryotic cell, a mammalian or a plant cell.

The transfection procedure using a compound of this invention can be carried out according to methods known per se. For transfecting a cell, e.g., with DNA or RNA, the +/- charge ratio between the positively charged compound of formula I and the negatively charged DNA or RNA is in the range of 0.1 to 50, more preferably from 0.1 to 10.

The transfection may be carried out in the presence of a helper lipid, a short chain phospholipid and/or another known transfection competent molecule. Examples of helper lipids are phospholipids, such as phosphatidylcholine or phosphatidylethanolamines or mixtures thereof. Preferred helper lipids are phosphatidylethanolamines, such as dioleoylphosphatidylethanolamine. Examples of short chain phospholipids are phosphatidylcholines that carry two C₆₋₁₂ fatty acid residues. Preferred short chain phospholipids are dicapryl- and dicapryloyl phosphatidylcholine. The helper lipid and/or short chain phospholipid is suitably in the form of a liposome, mixed micelle, organic solution, or aqueous dispersion.

Examples of transfection competent molecules include cationic lipids as described by J.B. Behr in Bioconjugate Chem. 5:382-389 (1994) and X. Gao and L. Huang in Gene Ther. 2:710-722 (1995); polycations as described by A.V. Kabanov and V.A: Kabanov in Bioconjugate Chem. 6:7-20 (1995); peptides and polymers and other nonviral gene delivery systems as described by F.D. Ledley in Human Gene Therapy 6:1129-1144 (1995). Other transfection competent molecules are those described by Legendre et al. (EP-A-95 118 338.3 and EP-A-96 100 603.8).

For transfection, an appropriate amount of at least one compound of formula I, suitably as an aqueous or organic solution, or in form of liposomes, mixed micelles or micelles, is added to an aqueous solution of the molecule to be transfected (e.g., plasmid DNA) or vice versa. Optionally, a cationic lipid or any other transfection-competent molecule, a helper lipid and, if desired, a short chain phospholipid is then added, either as an aqueous solution or dispersion, as an organic solution or dispersion, or in form of liposomes, mixed micelles or micelles. Alternatively, the molecule to be transfected may be added to a composition comprising at least one compound in accordance with this invention and, if desired, a cationic lipid or any other transfection-competent molecule, a helper lipid and a short chain phospholipid or vice versa. Suitably, the composition is an aqueous or organic solution, an aqueous or organic dispersion, or a liposome, a mixed micelle or a micelle. The final composition may be in liquid, solid (freeze-dried, controlled evaporative-dried), semisolid or aerosol form.

The optimal ratio between the components, i.e., the compound of formula I, the molecule to be transfected and, optionally, additional constituents, depends on the cell to be transfected. The optimal +/- charge ratio between the compound of formula I and the molecule to be transfected varies between 0.1-50, preferably between 0.1-10. The optimal molar ratio between the compound of formula I and additional constituents such as a cationic lipid, and/or another transfection competent molecule, and/or a helper lipids and/or a short chain phospholipid is 0.1-50, more preferably 0.1 to 10.

For transfecting cells in an animal or human patient the composition can be administered by oral, parenteral (i.v., i.m., s.c., i.d., i.p.) transdermal, pulmonary, nasal, rectal, ocular, ventricular, vascular (catheter) and intratumoral route. Furthermore, the composition can be administered by high velocity impaction administration to the skin surface. The progress of transfection can be measured by appropriate testing protocols which are known to those skilled in the art.

In another aspect, this invention relates to compositions comprising at least one compound of formula I, and optionally, a cationic lipid or any other known transfection competent molecule, a helper lipid and/or a short chain phospholipid, and optionally, a polynucleotide or any other anionic macromolecule.

The following examples which are not delimiting illustrate the invention further.

### Example 1

### Preparation of a compound of formula I wherein R¹ is an integrin bindung peptide and A is Dab-Dab-NHNH₂

The synthesis of compound of formula II (RGD-(Dab)₂NHNH₂) Gly-Gly-Cys-Arg-Gly-Asp-Met-Phe-Gly-Cys-Gly-Gly-Dab-Dab-N2H3 (II) was performed on a Peptide Synthesizer SP 650 (Labortec AG) using Wang resin by a FMOC protocol as described in Gene Therapy (1995), 2, 552-554. The peptide was cleaved from the resin by hydrazinolysis. The resulting product was deprotected and subjected to oxidative cyclization to yield the compound of formula II.

The homogeneity of the purified peptide was confirmed by analytical RP-HPLC and the molecule weight was determined by ISP MS: 1329.5 [M+H]⁺ calc. for C₅₀H₈₁N₂₁O₁₆S₃ (1328.5)

### Example 2

10 µg of plasmid DNA (pGL3-CMV) is diluted with 250 µl of 10 mM Tris Cl buffer pH 8.5. Then an appropriate amount of the compound obtained in Example 1, RGD-(Dab)₂NHNH₂, is mixed with the diluted DNA so that the +/- charge ratio between RGD-(Dab)₂NHNH₂ and DNA is 2/1. An appropiate volume of the resulting mixture containing 5 µg of plasmid DNA is then added per well of CaCo-2 cells grown in 6-well plate. 24h later, luciferase activity is measured. Results are shown in Table 1.

### Example 3

10 µg of plasmid DNA (pGL3-CMV) is diluted with 250 µl of 10 mM Tris Cl buffer pH 8.5. Then an appropriate amount of the compound obtained in Example 1, RGD-(Dab)₂NHNH₂, is mixed with the diluted DNA so that the +/- charge ratio between RGD-(Dab)₂NHNH₂ and DNA is 2/1. Then, dioleoyl phosphatidylethanolamine (DOPE) dispersion is added to the mixture so that the molar ratio between DOPE and RGD-(Dab)₂NHNH₂ is 5/1. An appropiate volume of the resulting mixture containing five µg of plasmid DNA is then added per well of CaCo-2 cells grown in 6-well plate. 24h later, luciferase activity is measured. Results are shown in Table 1.

**Table 1**

| | **Transfecting agent** | **Luciferase activity (relative LU) per well** |
|---|---|---|
| example 2 | RGD-(Dab)₂NHNH₂ | 503 |
| example 3 | RGD-(Dab)₂NHNH₂ + DOPE | 11412 |

### Example 4

7 nmoles of the compound obtained in Example 1, RGD-(Dab)₂NHNH₂, is mixed with 11 nmoles of dioleoyl-melittin, (DO-melittin, EP-A-96 100 603.8). Then, the mixture is added to 10 µg of plasmid DNA pGL3-CMV in a total volume of 200 µl 10 mM Tris Cl buffer pH 8.5. This corresponds to a +/-charge ratio between the cationic charge of the two transfection agents and the anionic charge of the DNA of 4/1. An appropiate volume of the resulting mixture containing 5 µg of plasmid DNA is then added per well of CaCo-2 cells grown in 6-well plate. 24h later, luciferase activity is measured. Table 2 shows the transfection efficiency of the RGD-(Dab)₂NHNH₂/DO-melittin mixture along with that of RGD-(Dab)₂NHNH₂ and DO-melittin alone.

**Table 2**

| **Transfection agent** | **Luciferase activity (RLU)** |
|---|---|
| RGD-(Dab)₂NHNH₂* | 250 |
| DO-melittin* | 6 X 10⁴ |
| RGD-(Dab)₂NHNH₂/ DO-melittin | 4 X 10⁵ |

| | |
|---|---|
| * prepared according to example 4 | |

### Example 5

1.75 nmoles of the compound obtained in Example 1, RGD-(Dab)₂NHNH₂, is mixed with 5.25 nmoles of the lipopeptide Nγ-palmitoyl-D-(α,γ-diaminobutyryl)-L-(α,γ-diaminobutyric acid) hydrazide (Palm-(Dab)₂NHNH₂). Then, the mixture is added to 10 µg of plasmid DNA pGL3-CMV in a total volume of 200 µl 10 mM Tris Cl buffer pH 8.5. This corresponds to a +/- charge ratio between the cationic charge of the two transfection agents and the anionic charge of the DNA of 2/1. Then, dioleoyl phosphatidylethanolamine (DOPE) dispersion is added to the mixture so that the molar ratio between DOPE and the two transfection agents is 5/1. An appropiate volume of the resulting mixture containing five µg of plasmid DNA is then added per well of CaCo-2 cells grown in 6-well plate. 24h later, luciferase activity is measured. Table 3 shows the transfection efficiency of the RGD-(Dab)₂NHNH₂/Palm-(Dab)₂NHNH₂ mixture along with that of RGD-(Dab)₂NHNH₂ and Palm-(Dab)₂NHNH₂ alone.

**Table 3**

| **Transfection agent** | **Luciferase activity (RLU)** |
|---|---|
| RGD-(Dab)₂NHNH₂* | 1 x 10⁴ |
| Palm-(Dab)₂NHNH₂* | 1 x 10⁵ |
| RGD-(Dab)₂NHNH₂/Palm-(Dab)₂NHNH₂ | 1 x 10⁶ |

| | |
|---|---|
| * prepared according to example 5 | |

## Claims

1. alpha,gamma-Diaminobutyric acid (DAB) containing oligopeptide derivatives of formula
R'-NH-A I
wherein
R' is a cell recognition agent, a membrane permeabilizing agent, a subcellular localization agent or a masking agent;
A is an oligopeptide devoid of one amino group and containing 1 to 200 amino acids, wherein at least one of the amino acid is alpha,gamma-diaminobutyric acids (DAB) and the terminal carboxyl group of the oligopeptide is converted into an amide, lower alkyl amide, di-lower alkyl amide or hydrazide.

2. Compounds according to claim 1, wherein the cell recognizing agent is an antibody or antibody fragment, a hormone, a protein growth factor, a cytokine and/or a ligand targeted to a cell surface receptor.

3. Compounds according to claims 1 or 2, wherein the subcellular localization agent is a nuclear localization molecule capable of delivering a polynucleotide or an anionic macromolecule from the cytoplasm to the nucleus of the cell.

4. Compounds according to claims 1 to 3 wherein the membrane permeabilizing agent is a molecule that aids in the passage of a polynucleotide or an anionic macromolecule across a membrane.

5. Compounds according to claim 1 to 4, wherein the masking agent is a molecule capable of increasing the circulatory half-life of the polynucleotide or the anionic macromolecule.

6. Compounds according to anyone of claims 1 to 5 wherein the terminal carbonyl group of the oligopeptide is converted into a hydrazide.

7. The use of at least one compound of formula I according to claims 1 to 6 as carrier for the transfection of cells with a polynucleotide or any other anionic macromolecule.

8. The use according to claim 6 in the presence of a cationic lipid or any other transfection competent molecule.

9. The use according to anyone of claims 7 and 8 in the presence of a helper lipid.

10. The use according to anyone of claims claim 7 to 9 in the presence of a short chain phospholipid.

11. A composition comprising at least one compound of formula I according to anyone of claims 1 to 6, and optionally a helper lipid, a short chain phospholipid and/or another transfection competent molecule.

12. A composition as in claim 11, wherein the components are in the form of an aqueous or organic solution, an aqueous or organic dispersion, or a liposome or a micelle.

13. A composition as in claims 11 or 12 wherein the composition is in solid, liquid, semisolid or aerosol form.

14. A composition as in claims 11 to 13 comprising a polynucleotide or any other anionic macromolecule.
